# EUROPEAN PATENT APPLICATION

(11) **EP 4 643 917 A1**
(43) Date of publication of application: **05.11.2025**
(21) Application number: 23909919.5
(22) Date of filing: 30.11.2023
(51) Int. Cl.: A61M 25/00

(54) **ASPIRATION SYSTEM**

(30) Priority: 28.12.2022 CN 202211697308
(71) Applicant: Lifetech Scientific (Shenzhen) Co., Ltd., Shenzhen, Guangdong 518063 (CN)
(72) Inventor: LIU, Quanzu, Shenzhen, Guangdong 518063 (CN); WU, Yufei, Shenzhen, Guangdong 518063 (CN)
(74) Representative: Michalski Hüttermann & Partner Patentanwälte mbB
(86) International application number: PCT/CN2023/135421
(87) International publication number: WO 2024/139989

(57) **Abstract**

An aspiration system (10), including an aspiration catheter (20), a catheter accessory (30), and an assembling component (40). The assembling component (40) includes an assembling base plate (300), and the aspiration catheter (20) and the catheter accessory (30) are detachably connected to the assembling base plate (300). The assembling base plate (300) comprises a catheter assembling area (310) for assembling the aspiration catheter (20). The aspiration catheter (20) includes a catheter seat (200) and a catheter body (100) which are connected to each other. The length of the catheter assembling area (310) is greater than the length of the aspiration catheter (20). The length of the catheter assembling area (310) of the assembling base plate (300) is greater than the length of the aspiration catheter (20), so that the aspiration catheter (20) can be fully straightened when fixed on the catheter assembling area (310), thereby preventing the assembled catheter body (100) from bending, ensuring the structural integrity of a guidewire cavity (121) and an aspiration cavity (111), and making an inner cavity of the aspiration catheter (20) less susceptible to collapse.

## Description

### Technical Field

The disclosure relates generally to medical devices, and more particularly to an aspiration system.

### Background

A thrombus is a small blood clot formed by bloodstream on a surface of an exfoliation part or a repair part of an endangium of a cardiovascular system, including insoluble fibrin, deposited platelets, accumulated white blood cells, and trapped red blood cells. At present, methods for treating thrombi mainly include a drug antithrombotic therapy and an artificial mechanical method for physically restoring vascular patency.

On one hand, the concentration of a thrombolytic and anticoagulant drug entering a blood vessel should not be too high, if not, it may cause side effects and toxicity to a human body. On the other hand, because the drug is discharged due to metabolism of the human body, the concentration of an antithrombotic drug in a blood vessel is low. Consequently, the antithrombotic drug for antithrombosis has slow action. The antithrombotic drug cannot be used to salvage acute thrombotic diseases such as acute myocardial infarction, cerebral vascular infarction, and acute deep vein thrombosis in lower limbs. For such diseases, physical thrombectomy can only be used. At present, there are thrombus aspiration catheters on the market that can slow down thrombi from blocking blood and restore blood supply, and have been widely used.

The axial length of the aspiration catheter is much greater than the diameter of an inner cavity of the aspiration catheter. The axial length is usually greater than one meter. Therefore, to compress the size of an aspiration system after packaging, the existing aspiration system is usually packaged by curling and coiling the aspiration catheter, so as to reduce the axial length of the aspiration catheter after packaging, thus facilitating transportation.

However, when the aspiration catheter is packaged by using the traditional curling and coiling method, the inner cavity of a catheter body may collapse in a long-term curled and coiled state, which affects the aspiration rate and passage of a guidewire, and in severe cases, may lead to failure of the product.

Therefore, a new technological approach is needed to solve the above-mentioned problems of the existing technology.

### Summary

The present disclosure aims to at least solve the problem that an inner cavity of an existing aspiration catheter easily collapses if the existing aspiration catheter is stored over time.

The present embodiments may provide an aspiration system, including an aspiration catheter, a catheter accessory, and an assembling component; the assembling component includes an assembling base plate, and the aspiration catheter and the catheter accessory are detachably connected to the assembling base plate; the assembling base plate includes a catheter assembling area for assembling the aspiration catheter; the aspiration catheter comprises a catheter seat and a catheter body which are connected to each other; and the length of the catheter assembling area is greater than the length of the aspiration catheter.

In the present disclosure, the length of the catheter assembling area of the assembling base plate is greater than the length of the aspiration catheter, so that the aspiration catheter can be fully straightened when fixed on the catheter assembling area, thereby preventing the assembled catheter body from bending, ensuring the structural integrity of a guidewire cavity and an aspiration cavity, and making the inner cavity of the aspiration catheter less susceptible to collapse.

In addition, the aspiration catheter according to the present invention may further have the following additional technical features:
Additionally or alternatively to any example described herein, the catheter assembling area is provided with a catheter fixing component for fixing the aspiration catheter; the catheter fixing component includes a fixed casing; and the catheter body is threaded into the fixed casing.

Additionally or alternatively to any example described herein, the fixed casing is in the shape of a straight tube and is disposed in a length direction of the catheter assembling area; and the inner diameter of the fixed casing is greater than the outer diameter of the catheter body.

Additionally or alternatively to any example described herein, the catheter fixing component further comprises a casing fixing member for clamping the fixed casing; the casing fixing member includes two casing fasteners respectively for fastening two end portions of the fixed casing; and inclination directions of the two casing fasteners are opposite.

Additionally or alternatively to any example described herein, the catheter seat is disposed at a proximal end of the catheter body; the catheter fixing component further includes a catheter seat fixing member for clamping the catheter seat; the casing fixing member further includes at least one casing supporting member disposed at a middle portion of the fixed casing and configured to support the fixed casing; the catheter seat fixing member is disposed at one end of the fixed casing;
Additionally or alternatively to any example described herein, the catheter seat includes a guidewire seat connected to the catheter body, and an aspiration seat connected to the catheter body; the catheter seat fixing member includes an aspiration fixing member for clamping the aspiration seat, and a guidewire fixing member for clamping the guidewire seat; and the aspiration fixing member and the guidewire fixing member are respectively inclined towards a middle portion of the catheter seat.

Additionally or alternatively to any example described herein, the assembling base plate further includes an accessory assembling area for mounting the catheter accessory; and the catheter assembling area and the accessory assembling area are respectively located on two sides along a length direction of the assembling base plate.

Additionally or alternatively to any example described herein, the catheter accessory includes a negative pressure source for providing a negative pressure, and a connection tube component connected between the aspiration catheter and the negative pressure source; and the accessory assembling area includes a connection fixing component for fixing the connection tube component, and a negative pressure fixing component for fixing the negative pressure source.

Additionally or alternatively to any example described herein, the connection tube component includes an extension tube connected to the aspiration catheter; the connection fixing component comprises at least two extension fixing members for clamping the extension tube; the at least two extension fixing members are disposed in the length direction of the assembling base plate in sequence; and inclination directions of the two extension fixing members are opposite.

Additionally or alternatively to any example described herein, the connection tube component further includes a conduction valve and a flow adjustment mechanism which are disposed on the extension tube; the connection fixing component further includes a valve body fixing member for clamping the conduction valve and an adjustment fixing member for clamping the flow adjustment mechanism; and the valve body fixing member is disposed between the catheter assembling area and the extension fixing member.

Additionally or alternatively to any example described herein, the adjustment fixing member is disposed between the two extension fixing members; and the flow adjustment mechanism is disposed between the adjustment fixing member and one of the two extension fixing members.

Additionally or alternatively to any example described herein, the negative pressure fixing component includes a negative pressure sleeve and a negative pressure clamp which are disposed on the assembling base plate; one end of the negative pressure source is clamped to the negative pressure clamp; and the other end of the negative pressure injection source is inserted into the negative pressure sleeve.

Additionally or alternatively to any example described herein, the negative pressure source includes a negative pressure injector; the negative pressure injector includes an injection tube and a piston rod movably connected to the injection tube; the injection tube is provided with a grip; a push plate is disposed at a tail end of the piston rod; the negative pressure sleeve is provided with a through hole for allowing the injection tube to pass through; and the negative pressure clamp is disposed between the grip and the push plate.

Additionally or alternatively to any example described herein, the catheter accessory includes a collection component for collecting waste liquid; the accessory assembling area includes a collection fixing component for fixing the collection component; the collection fixing component includes a connection assembling hole provided in the assembling base plate, and a connection strap passing through the connection assembling hole; and the width of the connection strap is greater than one-fifth of the width of the collection component.

Additionally or alternatively to any example described herein, the collection component includes a filter net basket and a waste liquid box; the waste liquid box comprises a box body, a box cover disposed on the box body, and a baffle plate disposed at an opening in an end portion of the box body; at least one liquid injection hole is provided in the baffle plate; the filter net basket is tubular; a filter net is disposed at a bottom of the filter net basket; and a handle is disposed on a side surface of the filter net basket.

Additionally or alternatively to any example described herein, step plates are disposed at edges of two sides of the assembling base plate; and the vertical distance from an edge of each step plate to the assembling base plate is at least greater than the thickness of the aspiration catheter.

### Brief Description of the Drawings

FIG. 1 is a schematic diagram of the entire structure of an aspiration catheter in Embodiment I;
FIG. 2 is a schematic diagram of the cross-sectional structure of the aspiration catheter in Embodiment I;
FIG. 3 is a side view of a supporting unit in Embodiment I;
FIG. 4 is a schematic diagram of the cross-sectional structure of the aspiration catheter with a bourdon tube in Embodiment I;
FIG. 5 is a side view of a supporting unit with a bourdon tube in Embodiment I;
FIG. 6 is a schematic diagram of the cross-sectional structure of an aspiration catheter with a sunken portion in Embodiment I;
FIG. 7 is a schematic diagram of a structure in which an aspiration cavity is elliptical in Embodiment I;
FIG. 8 is a schematic diagram of the structure of a distal end of an aspiration catheter in Embodiment I;
FIG. 9 is a schematic diagram of an internal structure of a catheter seat in Embodiment I;
FIG. 10 is a schematic diagram of the structure of a stress buffering seat in Embodiment I;
FIG. 11 is a schematic diagram of the structure of another implementation of a stress buffering seat in Embodiment I;
FIG. 12 is a schematic diagram of the entire structure of an aspiration system in Embodiment I;
FIG. 13 is a schematic diagram of the entire structure of a catheter accessory in Embodiment II;
FIG. 14 is a schematic diagram of the structure of an extension tube in Embodiment II;
FIG. 15 is a schematic diagram of the structure of a negative pressure injector in Embodiment II;
FIG. 16 is a schematic diagram of the structure of a waste liquid box in Embodiment II;
FIG. 17 is a schematic diagram of the structure of a filter net basket in Embodiment II;
FIG. 18 is a schematic diagram of the structure of a filter member in Embodiment II;
FIG. 19 is a schematic diagram of the entire structure of an assembling base plate in Embodiment II;
FIG. 20 is a schematic diagram of the assembled structure of an assembling base plate in Embodiment II.

### Detailed Description of the Invention

The embodiments of the present disclosure will be described in more detail below with reference to the accompanying drawings. Although the accompanying drawings show the exemplary embodiments of the present disclosure, it should be understood that the present disclosure can be implemented in various forms, and should not be limited to the embodiments stated herein. On the contrary, these embodiments are provided for understanding the present disclosure more thoroughly, and can completely transfer the scope of the present disclosure to those skilled in the art.

It should be understood that the terms used herein are only for the purpose of describing specific exemplary embodiments and are not intended to be restrictive. Unless otherwise indicated, the singular forms such as "a/an", "one", and "the" used herein can also indicate a plural form. Although the terms first, second, third, etc. may be used herein to describe multiple elements, components, areas, layers, and/or sections, these elements, components, areas, layers, and/or sections should not be limited by these terms. These terms can only be used to distinguish one element, component, area, layer, or section from another element, component, area, layer, or section.

For ease of description, spatial relative relationship terms can be used herein to describe a relationship between an element or feature shown in the figure relative to another element or feature, such as "internal", "external", "inner side", "outer side", "beneath", "below", "on", and "above". This spatial relative relationship term means including different orientations of a device in use or operation, in addition to the orientations depicted in the figures.

For ease of description, the following description uses the terms "proximal" and "distal". "proximal" means positions close to an operator, and "distal" means positions far away from the operator. The phrase "axial direction" should be understood as indicating a direction in which an intervention element is pushed in or out, and a direction perpendicular to the "axial direction" is defined as a "radial direction".

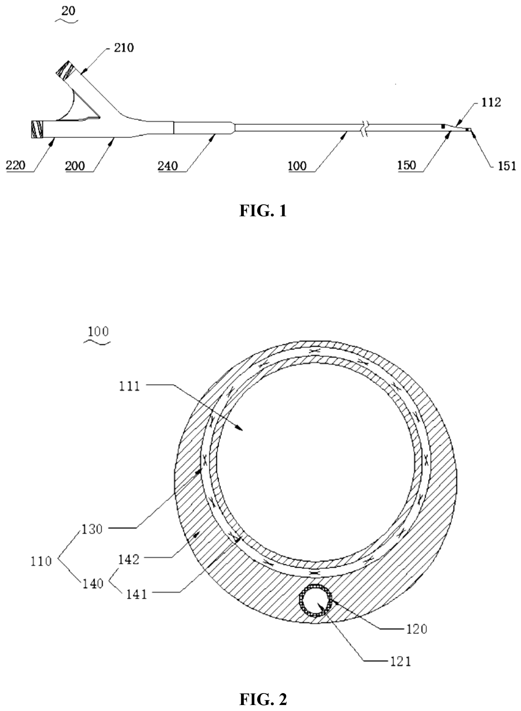

Embodiment I: Embodiment I of the present disclosure provides an aspiration catheter 20. The structure of the aspiration catheter 20 is shown in FIG. 1. The aspiration catheter 20 includes a catheter body 100 and a catheter seat 200 connected to the proximal end of the catheter body 100. The catheter body 100 includes an aspiration tube 110 and a guidewire tube 120. The aspiration tube 110 and the guidewire tube 120 can be fixedly connected after being respectively formed, and the aspiration catheter 20 can alternatively be integrally formed with the guidewire tube 120. As shown in FIG. 2, a hollow aspiration cavity 111 is formed inside the aspiration tube 110. The aspiration cavity 111 is configured to aspirate a thrombus. A hollow guidewire cavity 121 is formed inside the guidewire tube 120, and the guidewire cavity 121 is configured to allow a guidewire to pass through. In this embodiment, the hardness of the catheter body 100 gradually decreases from the proximal end to the distal end.

Since the distal portion of the catheter body 100 has low hardness, the catheter body 100 has good passability when passing through a curved blood vessel. A part which is in contact with an inner wall of the blood vessel of the distal portion of the catheter body 100 is softer than the other part, thus causing less irritation to the inner wall of the blood vessel during pushing, and making it less likely to cause secondary damage to the inner wall of the blood vessel.

Meanwhile, since the proximal portion of the catheter body 100 has high hardness, connection portions of the catheter body 100 and the catheter seat 200 has high connection strength, which can ensure good pushing performance. Moreover, it can ensure that a part that is connected to the catheter seat 200 of the catheter body 100 does not bend easily, thus avoiding narrowing of the aspiration cavity 111 or the guidewire cavity 121. Therefore, it can ensure that the aspiration area of the aspiration cavity 111 provides smooth passage of the guidewire through the guidewire cavity 121, thereby ensuring the aspiration rate and the surgical efficiency.

The present disclosure uses two cavities, made up of the aspiration tube 110 and the guidewire tube 120. Compared with a single cavity in the existing technology, the aspiration catheter 20 of the present disclosure does not require use of a dilator during use, and it is more convenient to thread the guidewire, which can improve the surgical efficiency and the success rate of surgery. Before the aspiration catheter 20 enters the blood vessel, the guidewire is first threaded into the blood vessel to establish a pathway for the aspiration catheter 20 to enter the blood vessel. Then, the aspiration catheter 20 is threaded into the blood vessel along the pathway established by the guidewire and reaches a predetermined position.

The aspiration tube 110 includes a supporting unit 130 and a sealing unit 140 disposed on the supporting unit 130. The hardness of the supporting unit 130 and/or the sealing unit 140 gradually decreases from the proximal end to the distal end.

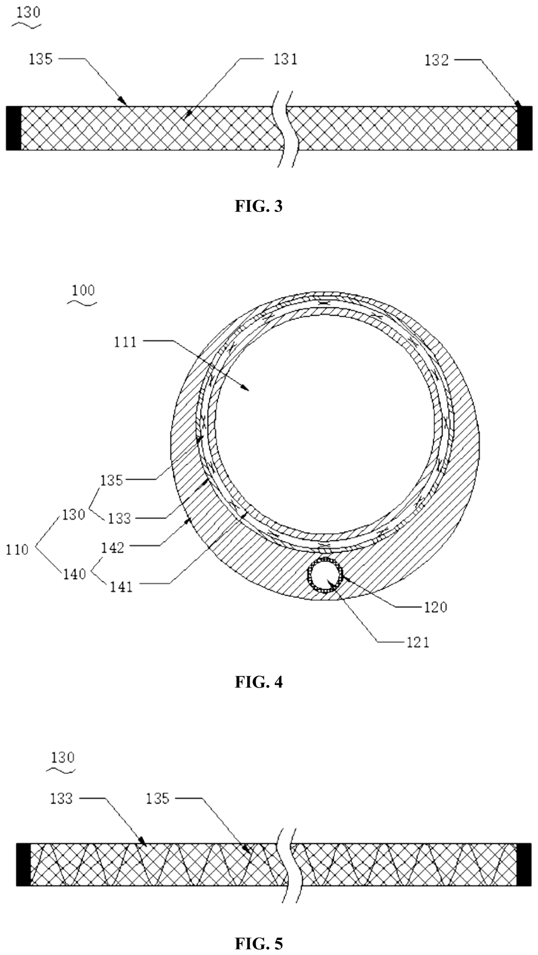

As shown in FIG. 3, the supporting unit 130 includes a braided tube 135 made by spirally winding a braiding wire 131, and the two ends of the braiding wire 131 are fixed and closed by pressure rings 132. The braiding wire 131 is a nickel titanium wire or a stainless steel wire. The sealing unit 140 is a thin tube made of a polymer material, and the sealing unit 140 seals pores of the supporting unit 130 through thermal treatment.

In this embodiment, the braided tube 135 is formed by braiding a plurality of strands of braiding wires 131. There may be 8, 12, or 16 strands of braiding wires 131, which are selected depending on an actual need. The hardness of the supporting unit 130 decreases in sequence from the proximal end to the distal end. After the braiding wire 131 is braided into a tubular structure, a mesh-like braided tube 135 is formed through thermal setting treatment. The supporting unit 130 braided with the braiding wire 131 has good bending resistance, which can ensure good compliance, avoid the impact on an area of the aspiration cavity 111 due to bending, ensure the aspiration rate, and ensure good pushing performance.

In an embodiment, the braiding density of the braiding wire 131 of the braided tube 135 decreases in sequence from the proximal end to the distal end, or the wire diameter of the braiding wire 131 of the braided tube 135 decreases in sequence from the proximal end to the distal end, so that the hardness of the supporting unit 130 gradually decreases from the proximal end to the distal end.

As shown in FIG. 4 and FIG. 5, in other embodiments, the supporting unit 130 may alternatively include a spring tube 133. The supporting unit 130 can be formed by the braided tube 135 alone, or by combining the braided tube 135 with the spring tube 133. The supporting unit 130 with the spring tube 133 can further improve its bending resistance, and can have compliance and resilience, thus improving the surgical efficiency.

If the supporting unit 130 includes the braided tube 135 and the spring tube 133, during manufacture, the spring tube 133 first surrounds a core rod of a mold, and then the braided tube 135 is braided on an outer side of the spring tube 133 through the braiding wire 131. Alternatively, after the braided tube 135 is braided, the spring tube 133 surrounds an outer side of the braided tube 135.

In an embodiment, the spring pitch of the spring tube 133 increases in sequence from the proximal end to the distal end, or the wire diameter of the spring tube 133 decreases in sequence from the proximal end to the distal end, so that the hardness of the bourdon tube 133 decreases in sequence from the proximal end to the distal end.

As shown in FIG. 2, in one embodiment, the sealing unit 140 includes an inner sealing tube 141 disposed on an inner side of the supporting unit 130, and an outer sealing tube 142 disposed on an outer side of the supporting unit 130. The inner sealing tube 141 and the outer sealing tube 142 tightly abut against and are fixed to the inner and outer sides of the supporting unit 130 through thermal treatment respectively, to seal the supporting unit 130. Both the inner sealing tube 141 and the outer sealing tube 142 are made of polymer materials. For example, the inner sealing tube 141 is a polytetrafluoroethylene (PTFE) thin tube, and the outer sealing tube 142 is a Pebax or nylon thin tube.

The inner sealing tube 141 is closed and enclosed to form the aspiration cavity 111, and the guidewire tube 120 is fixed on an inner or outer wall of the aspiration tube 110. The guidewire tube 120 can be integrally formed with the aspiration tube 110, or the guidewire tube 120 and the aspiration tube 110 are separately formed and are then fixedly connected to each other by hot melting.

The hardness of the sealing unit 140 decreases in sequence from the proximal end to the distal end. For instance, the material hardness of the inner sealing tube 141 and/or the outer sealing tube 142 decreases in sequence from the proximal end to the distal end, and the material hardness changes in a range from 30D to 90D. Or, the material thickness of the inner sealing tube 141 and/or the outer sealing tube 142 decreases in sequence from the proximal end to the distal end.

In one embodiment, the inner sealing tube 141 uses an inner membrane made of a material with a good thermal stability, such as PTFE and a polyimide (PI) material. The melting point of the outer sealing tube 142 is lower than the melting point of the inner sealing tube 141. The outer sealing tube 142 uses an outer membrane made of a material with a melting point higher than the sterilization temperature (115°C), and the outer membrane may be polyurethane, PEBAX, nylon, a polyethylene material, or the like.

In addition, the melting point of the guidewire tube 120 is greater than the melting point of the outer sealing tube 142 and the inner sealing tube 141, and the hardness of the guidewire tube 120 is less than both the outer and inner layer of the braided tube 135. If the guidewire tube 120 is too thick, the guidewire tube will be broken easily during the pulling process. If the guidewire tube 120 is too thin, the guidewire tube cannot be stretched easily, and will affect the wall thickness of the entire catheter body 100. Therefore, in one embodiment, the thickness of the guidewire tube 120 is set to 0.02 mm to 0.1 mm, to ensure that the guidewire tube 120 has a sufficient flexibility.

The outer surface of the guidewire tube 120 is a rough outer surface by surface treatment. In one embodiment, the contact angle of the guidewire tube 120 is 50° to 80° after using surface treatment, so that the guidewire tube 120 can be better bonded to the outer sealing tube 142 or the inner sealing tube 141.

The thickness of the outer sealing tube 142 is set to 0.1 mm to 0.5 mm, ensuring that the outer layer of the braided tube 135 has sufficient scratch resistance, and the entire catheter body 100 has a proper thickness, which makes that the outer sealing tube 142, does not occupy too much space.

In the present embodiment, the hardness of the supporting unit 130 and/or the sealing unit 140 decreases in sequence from the proximal end to the distal end, to reduce the hardness of the catheter body 100 in sequence from the proximal end to the distal end. Thus, the head of the distal portion of the catheter body 100 is softer than that of the proximal portion, which can more effectively prevent the catheter body 100 from scratching a curved blood vessel when passing through or located at the blood vessel, and provides better adhesion of the catheter body 100. The hardness of the proximal portion of the catheter body 100 is greater than the hardness of the distal portion of the catheter body 100, which improve the pushing and withdrawing properties of the catheter. Moreover, when the catheter body 100 bends under a force, the part of the catheter body 100 connected to the catheter seat 200 is more prone to bending under the force, which makes the aspiration cavity 111 collapse, and the aspiration rate is affected. A part connected to the catheter seat 200 of the proximal end of the catheter body 100 has higher hardness and can effectively avoid the problem of collapse of the aspiration cavity 111.

The guidewire tube 120 is buried in the outer sealing tube 142 of the catheter body 100, and the thickness of one side configured to bury the guidewire tube 120 of the outer sealing tube 142 is greater than the thickness of the other side, thereby providing an accommodating space for the guidewire tube 120. The wall thickness of the outer sealing tube 142 of one side is less than the wall thickness of the other side having the guidewire tube 120, which can reduce the overall outer diameter of the catheter body 100, making it easier for the catheter body 100 to enter a blood vessel and ensuring an aspiration area of the aspiration tube 110.

As shown in FIG. 2, in an embodiment, when the aspiration catheter 20 is applied to a blood vessel with a diameter greater than 5 mm, the guidewire tube 120 is spaced apart from the aspiration tube 110, and the guidewire tube 120 is fixed inside the outer sealing tube 142 by hot melting, which makes the inner cavity of the aspiration tube 110 circular. Through the above technical solution, the present embodiment can simultaneously ensure the integrity of the form of the aspiration cavity 111 and the wall strength of the catheter body 100, thereby ensuring the integrity of the catheter body 100 during passage through a curved blood vessel and preventing the aspiration cavity 111 of the aspiration tube 110 from collapsing inwards during passage through a curved portion of the blood vessel. Thus, it ensures the aspiration rate of the aspiration catheter 20 and improves the success rate of the surgery.

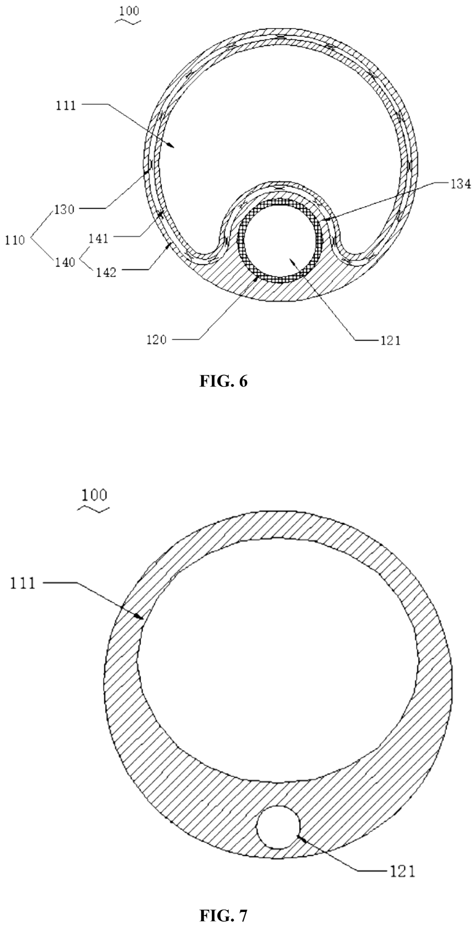

As shown in FIG. 6, in other embodiments, when the aspiration catheter 20 is applied to a blood vessel with a diameter less than 5 mm, the supporting unit 130 of the aspiration catheter 110 is sunken inwards to form a receiving portion 134, and the guidewire tube 120 is disposed in the receiving portion 134 and is fixed to the outer sealing tube 142 by hot melting. Due to the small area of the aspiration cavity 111 of the aspiration catheter 20 when the aspiration catheter 20 aspirates a thrombus from a small branch vessel of an endovascular lumen, the guidewire tube 120 is placed in the receiving portion 134 to ensure the areas of both the aspiration cavity 111 and the guidewire cavity 121, thus improving the aspiration efficiency and ensuring the success rate of surgery.

In addition, the guidewire tube 120 is disposed in the receiving portion 134, which not only prevents the catheter body 100 from scratching the vascular wall when passing through the curved blood vessel, but also prevents the guidewire tube 120 from becoming narrow due to bending. This ensures the surgical efficiency and the success rate of surgery. Meanwhile, the contact area between the guidewire tube 120 and the aspiration tube 110 becomes larger, making the connection more secure.

As shown in FIG. 7, in other embodiments, the aspiration cavity 111 of the aspiration tube 110 can be further set to be elliptical to enable the catheter body 100 to obtain a larger aspiration area, thus improving the aspiration rate.

In other embodiments, the guidewire tube 120 may also be disposed on an inner wall of the aspiration tube 110 or on an outer wall of the aspiration tube 110.

After the guidewire tube 120 and the aspiration tube 110 are formed separately, the guidewire tube 120 and the outer sealing tube 142 are fixed by hot melting, which makes the guidewire tube 120 fixed on an outer side wall of the aspiration tube 110 through hot melting. Since the guidewire tube 120 does not occupy the aspiration area of the aspiration tube 110, the area of the aspiration cavity 111 inside the aspiration tube 110 and the aspiration rate are ensured. After the guidewire tube 120 and the aspiration tube 110 are formed separately, the guidewire tube 120 and the inner sealing tube 141 are fixed by hot melting, which makes the guidewire tube 120 fixed on an inner side wall of the aspiration tube 110 through hot melting. Thus, the guidewire tube 120 is disposed in the aspiration tube 110, without increasing the outer diameter of the entire catheter body 100, and the separate arrangement of the aspiration tube 110 and the guidewire tube 120 is achieved, which ensures smooth guidewire pushing.

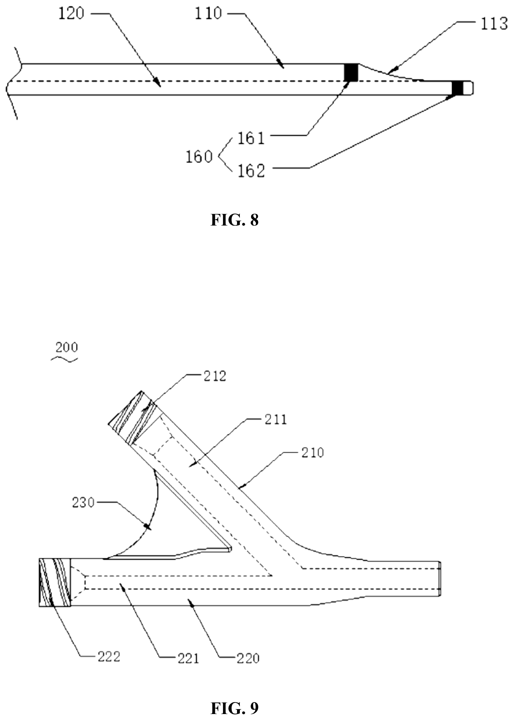

As shown in FIG. 1 and FIG. 8, a guide head 150 is disposed at a distal direction of the catheter body 100, and an aspiration port 112 is provided in a side surface of the aspiration tube 110. The guide head 150 is disposed at a distal direction of the guidewire tube 120, and the guide head 150 is located at a distal direction of the aspiration port 112. A head cavity 151 communicated to the guidewire cavity 121 is disposed in the guide head 150. The guidewire is threaded into the head cavity 151 from the guidewire cavity 121 and exits from the head cavity 151. Since the guide head 150 is disposed at the distal direction of the catheter body 100, it is possible to better guide the aspiration catheter 20 to pass through a curved blood vessel or a venous valve. The hardness of the guide head 150 is less than the hardness of the catheter body 100, which can prevent the catheter body 100 from scratching the vascular wall or the venous valve when the catheter body 100 passes through the curved blood vessel.

In this embodiment, the material of the guide head 150 is a polymer material with a hardness of 80A to 40D, such as Pebax, silica gel, or nylon, which can be selected according to an actual need.

The aspiration port 112 is provided in a side surface of the aspiration tube 110, and the aspiration port 112 is provided in a distal end portion of the aspiration tube 110. An end surface of the aspiration port 112 is inclined relative to the axial direction of the aspiration tube 110. Thrombi are usually intensively attached on vascular walls, and especially for curved blood vessels, the thrombi are usually intensively attached at curved portions of the blood vessels. Therefore, it is difficult for the existing thrombus aspiration catheter to completely remove the thrombi attached to the curved blood vessels during thrombus aspiration. As a result, there are still thrombi remaining after the aspiration, and there is a risk of re-embolization of downstream branch vessels.

In the present embodiment, the aspiration port 112 is provided towards the side surface of the aspiration tube 110, and the aspiration port 112 is inclined relative to the axial direction of the aspiration tube 110, to provide a higher aspiration force for eccentric thrombi and mural thrombi, to ensure the aspiration effect. Especially for the thrombi located at the curved blood vessels, the aspiration port 112 can directly face the thrombi attached to the curved blood vessels, thereby more thoroughly clearing the thrombi at the curved blood vessels and avoiding residues. Moreover, the inclined aspiration port 112 increases the aspiration area, thereby providing a higher aspiration force under the same aspiration equipment, and ensuring the clearing effect on thrombi.

A sunken portion 113 is disposed on an end surface of the aspiration port 112, and the sunken portion 113 is connected to a tube cavity of the aspiration tube 110. In this embodiment, the sunken portion 113 is sunken inwards towards an inner side of the end surface of the aspiration port 112, and the sunken portion 113 is arc-shaped.

For eccentric thrombi, mural thrombi, and thrombi located at curved blood vessels, due to their unique morphologies and positions, it is often hard to completely clear these thrombi. In the present embodiment, the sunken portion 113 is disposed on the end surface of the aspiration port 112, which makes the aspiration port 112 more in line with the shape of a thrombus, and further enlarges an opening area of the aspiration port 112, to increase the aspiration force, thereby ensuring that the thrombus can be cleared more thoroughly and avoiding the risk of embolization caused by detachment of an uncleared thrombus.

The catheter body 100 is provided with an imaging portion 160. The imaging portion 160 is disposed at the aspiration port 112. The imaging portion 160 is configured to indicate the distal end position of the catheter body 100 and the orientation of the aspiration port 112. Meanwhile, the imaging portion 160 can be further configured to fix the braiding wire 131 at the end portion of the supporting unit 130. The imaging portion 160 includes a first imaging element 161 disposed at a proximal end portion of the aspiration port 112, and a second imaging element 162 disposed at a distal end portion of the aspiration port 112. The first imaging element 161 and the second imaging element 162 may be imaging rings surrounding the catheter body 100 or imaging points embedded on the catheter body 100. Specifically, the first imaging element 161 and the second imaging element 162 are made of a material with an imaging function, such as platinum or tantalum metal, and are of ring-like or dot-like structures.

In this embodiment, the first imaging element 161 is an imaging ring that surrounds the aspiration tube 110 and is located at a proximal end of the aspiration port 112, and the second imaging element 162 is an imaging ring that surrounds the guide head 150 and is located at a distal end of the aspiration port 112. Since the guide head 150 is disposed at the distal end of the guide tube 120, and the guide tube 120 is disposed on an inner wall of the aspiration tube 110, the axial position of the second imaging element 162 surrounding the guide head 150 is different from the axial direction of the first imaging element 161 surrounding the aspiration tube 110. Through the above structural design with the two imaging rings, the orientation of the aspiration port 112 can be determined according to the sizes and positions of the first imaging element 161 and the second imaging element 162 from an image such as a digital subtraction angiography (DSA), so as to adjust the orientation after aspiration before aspiration.

In other embodiments, the first imaging element 161 is an imaging point that is disposed on the aspiration tube 110 and is located at a proximal end of the aspiration port 112, and the second imaging element 162 is an imaging ring that surrounds the guide head 150 and is located at a distal end of the aspiration port 112. A projection of the first imaging element 161 on the cross section of the catheter body 100 and a projection of the second imaging element 162 on the cross section of the catheter body 100 are misaligned, which allows a doctor to more clearly distinguish the axial position of the aspiration port 112 and the orientation of the sunken portion 113. To aspirate a thrombus at a curved blood vessel, if the sunken portion 113 is not aligned with the thrombus, the doctor can adjust the orientation of the aspiration port 112 in a timely manner to maximize the aspiration efficiency.

When the aspiration catheter 20 is placed into the blood vessel and the catheter body 100 passes through a curved portion of the blood vessel, the guide head 150 at the distal end of the catheter body 100 bends due to collision with the vascular wall. When the distance between the first imaging element 161 and the second imaging element 162 is shortened to 80% of an initial distance (i.e. the distance in a unpressed state), it indicates that the curvature of the blood vessel is too large, and there is a risk of puncturing an opposite vascular wall at the distal end of the guide head 150. The doctor needs to adjust the surgical method in a timely manner. The distance between the first imaging element 161 and the second imaging element 162 is the distance between two points closest to each other on the first imaging element 161 and the second imaging element 162.

As shown in FIG. 1 and FIG. 9, the catheter seat 200 includes an aspiration seat 210 for connecting the aspiration tube 110, and a guidewire seat 220 for connecting the guidewire tube 120. An aspiration connection cavity 211 is provided inside the aspiration seat 210. The aspiration connection cavity 211 communicates with the aspiration cavity 111 of the aspiration tube 110. A guidewire connection cavity 221 is provided inside the guidewire seat 220, and the guidewire connection cavity 221 communicates with the guidewire cavity 121 of the guidewire tube 120.

An aspiration connection port 212 is provided in an end portion of the aspiration seat 210. The aspiration connection port 212 is configured to communicate a negative pressure source 500, such as a negative pressure pump or a negative pressure injector. The negative pressure source provides a negative pressure for the aspiration catheter 20 to aspirate a thrombus, so that a thrombus in a blood vessel can be aspirated from the aspiration cavity 111. A guidewire connection port 222 is provided in an end portion of the guidewire seat 220, and the guidewire is threaded through the guidewire connection cavity 221 and the guidewire cavity 121 in sequence from the guidewire connection port 222, and exits from a distal end of the guidewire cavity 121.

The axial direction of the guidewire connection cavity 221 is the same as the axial direction of the guidewire cavity 121, or the angle between the axial direction of the guidewire connection cavity 221 and the axial direction of the guidewire cavity 121 is less than 15°. Through the above design, the pathway of the guidewire through the guidewire connection port 222, the guidewire connection cavity 221, and the guidewire cavity 121 is smoother, without a large curvature, making it easier to establish an enter pathway of the aspiration catheter 20.

The aspiration seat 210 is disposed on one side of the guidewire seat 220, and a holding portion 230 is disposed between the guidewire seat 220 and the aspiration seat 210. The holding portion 230 is flat, and an operator can grasp the catheter seat 200 through the holding portion 230. In this embodiment, the aspiration seat 210 and the catheter seat 200 are cylindrical. The guidewire seat 220 is coaxial with the catheter body 100. The aspiration seat 210 and the catheter seat 200 are set at a predetermined angle, which is 30 degrees to 60 degrees.

For eccentric thrombi, mural thrombi, and thrombi located at curved blood vessels, the thrombi are at different positions in different patients, so that after the aspiration catheter 20 is threaded into a blood vessel, an operator needs to further align the aspiration port 112 with a direction in which a thrombus is located. In a specific operation, the operator can grasp the catheter seat 200 through the holding portion 230 and rotate the catheter seat 200 around the guidewire seat 220 serving as an axis to drive the catheter body 100 to rotate, so that an opening of the aspiration port 112 faces the thrombus. The operator then performs targeted aspiration on the thrombus.

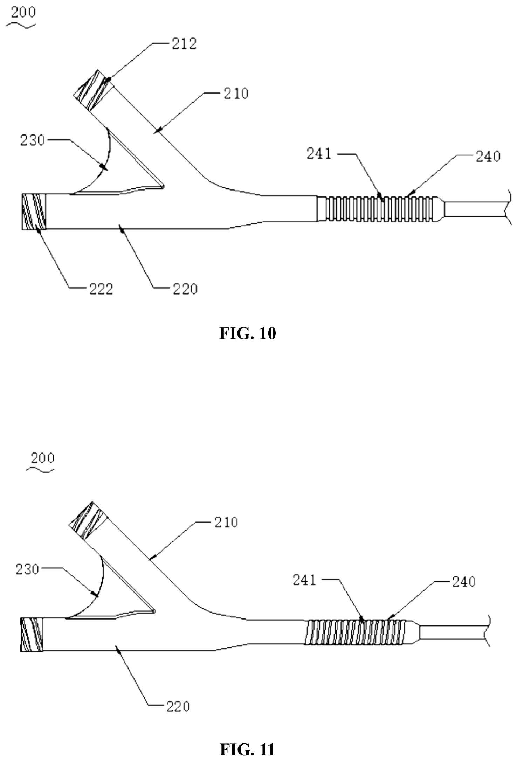

As shown in FIG. 1 and FIG. 10, a stress buffering seat 240 is disposed at the distal end of the catheter seat 200, and the stress buffering seat 240 surrounds the catheter body 100. The stress buffering seat 240 is elastic. The hardness of the stress buffering seat 240 is less than the hardness of the catheter seat 200 and is greater than the hardness of the catheter body 100. When the aspiration catheter 20 is placed into a human lumen, the catheter body 100 is inside the body and the catheter seat 200 is outside the body. When the catheter body 100 bends under a force, the part of the catheter body 100 connected to the catheter seat 200 is more prone to bending. If the catheter body 100 continues to bend under the force or the curvature is too large, the catheter body 100 is prone to plastic deformation, which affects the success rate of surgery. In addition, even if the catheter body 100 does not undergo the plastic deformation due to torsion, the aspiration cavity 111 inside the catheter body 100 is also prone to a decrease in its cross-sectional area due to excessive torsion, which affects the aspiration rate.

In the present embodiment , the connection strength for the catheter body 100 and the catheter seat 200 is enhanced by arranging the stress buffering seat 240 at the distal end of the catheter seat 200 and stress concentration at a connection between the catheter body 100 and the catheter seat 200 is reduced, and the inward collapse of the aspiration cavity 111 of the catheter body 100 is avoided, thereby ensuring the aspiration rate of the aspiration catheter 20 and improving the success rate of surgery.

A stress buffering ring 241 is disposed on the stress buffering seat 240. In this embodiment, the stress buffering ring 241 is disposed in the shape of an annular groove. A plurality of stress buffering rings 241 are disposed on the stress buffering seat 240, and the plurality of stress buffering rings 241 are spaced apart from each other. Therefore, when the catheter body 100 drives the stress buffering seat 240 to bend, the stress buffering rings 241 can further counteract a bending stress, to provide a deformation space for the stress buffering seat 240 and make the stress buffering seat 240 more uniform during bending under the force, thus further avoiding the inward collapse of the aspiration cavity 111 of the catheter body 100 and ensuring the aspiration rate.

As shown in FIG. 11, in other embodiments, the stress buffering ring 241 is arranged in the shape of a spiral convex ring. When it is necessary to make the opening of the aspiration port 112 face the position of an eccentric thrombus, the guidewire seat 220 needs to be rotated to make the catheter body 100 to rotate. The stress buffering ring 241, which is arranged in the shape of the spiral convex ring, can provide a reaction force for the catheter seat 200 to counteract the rotation direction, to reduce the torsional deformation of the stress buffering seat 240 and avoiding the plastic deformation of the catheter body caused by excessive rotation.

In summary, the aspiration catheter 20 of the present embodiment can adapt to an aspiration environment of a curved blood vessel, and the catheter body 100 has good passability and retractability. After the catheter body 100 passes through the curved blood vessel, both the aspiration tube 110 and the guidewire tube 120 does not collapse easily due to bending. Meanwhile, the aspiration catheter has a good aspiration effect on eccentric thrombi, mural thrombi, and thrombi located at curved blood vessels, thus avoiding the risk of re-embolization caused by residual thrombi after aspiration. In addition, during the adjustment of an aspiration posture of the catheter body 100, the connection strength for the catheter body 100 and the catheter seat 200 can also be guaranteed, and the catheter body 100 will not undergo plastic deformation due to torsion or bending, which ensures the success rate of surgery.

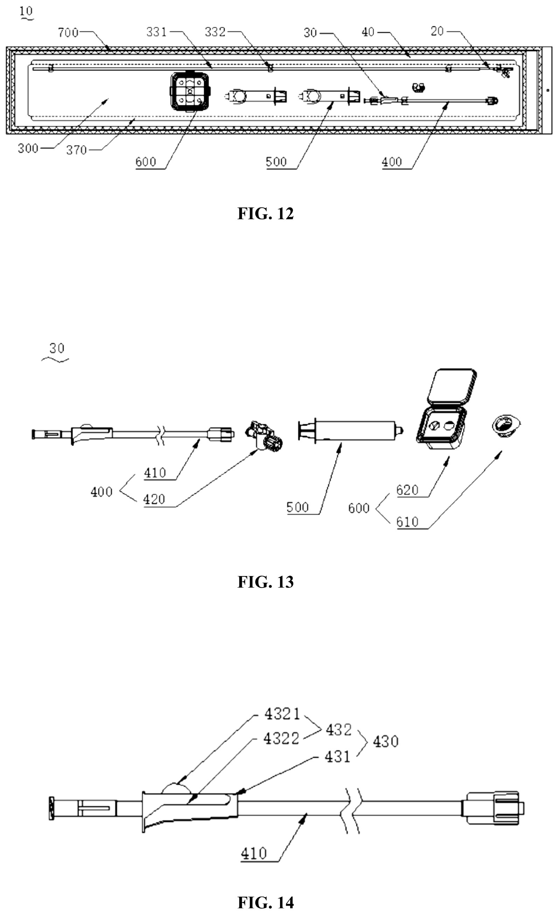

Embodiment II: Embodiment II of the present disclosure provides an aspiration system 10. As shown in FIG. 12, the aspiration system 10 includes an aspiration catheter 20, a catheter accessory 30, and an assembling component 40. The assembling component 40 includes an outer package 700 and an assembling base plate 300. The outer package 700 covers the assembling base plate 300, and the aspiration catheter 20 and the catheter accessory 30 are detachably connected to the assembling base plate 300 of the assembling component 40. The catheter accessory 30 includes a connection tube component 400, a negative pressure source 500, and a collection component 600.

As shown in FIG. 13 and FIG. 14, the connection tube component 400 includes an extension tube 410 and a conduction valve 420. One end of the extension tube 410 is configured to connect the aspiration connection port 212, and the other end of the extension tube 410 is configured to connect the conduction valve 420. The extension tube 410 is provided with a flow adjustment mechanism 430. The flow adjustment mechanism 430 is configured to adjust an aspiration flow during aspiration.

The flow adjustment mechanism 430 surrounds the extension tube 410. The flow adjustment mechanism 430 includes a valve body 431 surrounding the extension tube 410 and a control key 432 disposed on the valve body 431. The aspiration flow during the aspiration is adjusted by using the control key 432.

In this embodiment, the control key 432 includes a roller 4321 that is slidably connected to the valve body 431, and a sliding chute 4322 that is disposed in the valve body 431 and is axially inclined relative to the extension tube 410. The roller 4321 is slidably connected to the sliding chute 4322, and a side surface of the roller 4321 tightly abuts against the extension tube 410. Since the sliding chute 4322 is inclined relative to the extension tube 410, when the roller 4321 is driven to move along the sliding chute 4322, the pressure between the roller 4321 and the extension tube 410 can be adjusted, thereby reducing or increasing the inner diameter of the tube cavity of the extension tube 410 and adjusting the flow of the extension tube 410.

During the aspiration, an operator adjusts the flow according to the actual situation. In aspiration, the operator first adjusts the aspiration flow to a small aspiration flow and observes an aspiration situation. If a thrombus can be aspirated successfully from a blood vessel, there is no need to adjust the flow adjustment mechanism. If the thrombus cannot be aspirated smoothly at the small aspiration flow, the operator gradually increases the aspiration flow through the flow adjustment mechanism, thereby increasing the aspiration force of the aspiration catheter to smoothly aspirate the thrombus.

In the present disclosure, the aspiration catheter is controlled by gradually adjusting the aspiration force. At the beginning of the aspiration, a smaller aspiration force is used, causing a low negative pressure inside the blood vessel, which can better protect the blood vessel. During the aspiration process, if the low negative pressure cannot aspirate the thrombus, the aspiration force is gradually increased to use an appropriate aspiration force to aspirate the thrombus. In a case of ensuring that the thrombus can be aspirated, the blood vessel is protected as much as possible, to provide a guarantee for the surgical safety.

As shown in FIG. 13, the conduction valve 420 is connected between the extension tube 410 and the negative pressure source 500. The conduction valve 420 is configured to control the extension tube 410 to be opened or closed. The conduction valve 420 includes a straight-through valve, a three-way valve, or the like. In this embodiment, the conduction valve 420 is a straight-through valve. Before and after aspiration, the conduction valve 420 is closed to avoid blood loss of a patient due to a misoperation, and may improve the surgical safety.

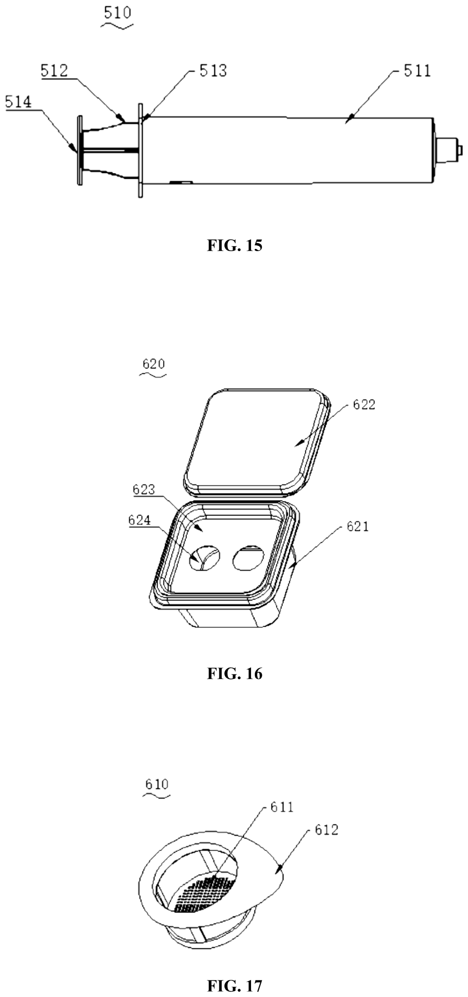

As shown in FIG. 13 and FIG. 15, the negative pressure source 500 includes a negative pressure pump, negative pressure injector 510, or the like. The negative pressure source 500 provides a negative pressure for the aspiration catheter 20 to aspirate a thrombus, so that the thrombus in a blood vessel is aspirated from the aspiration cavity 111. In this embodiment, the negative pressure source 500 is the negative pressure injector 510. The negative pressure injector 510 is used as the negative pressure source 500. Compared with the negative pressure pump, the negative pressure injector does not need to be connected to a power supply, and is flexible to operate.

The negative pressure injector 510 includes an injection tube 511 and a piston rod 512 movably connected to the injection tube 511. A grip 513 is disposed on a tube body of the injection tube 511, and a push plate 514 is disposed at the tail end of the piston rod 512. During operation, an operator holds the grip 513 on the injection tube 511, and then adjusts the position of the piston rod 512 inside the injection tube 511 by moving the push plate 514, thereby adjusting a negative pressure inside the negative pressure injector 510.

In an embodiment, it is necessary to discharge air in the aspiration catheter 20 before aspiration. First, the guidewire cavity 121 of the aspiration catheter 20 is rinsed with the injector containing physiological saline until liquid flows out of the guidewire cavity 121 at the tail end of the aspiration catheter 20, thus discharging the air in the guidewire cavity 121. Then, the straight-through valve and the flow adjustment mechanism 430 on the extension tube 410 are turned on, and the aspiration cavity 111 of the aspiration catheter 20 is rinsed with the injector containing the physiological saline until liquid flows out of the aspiration cavity 111 at the tail end of the aspiration catheter 20, thus discharging air in the aspiration cavity 111. After the air is discharged, the conduction valve 420 and the flow adjustment mechanism 430 are turned off. Finally, the negative pressure injector 510 is connected to the aspiration catheter 20.

The aspiration catheter 20 is placed into the blood vessel for thrombus aspiration. After confirmed by observation that the aspiration catheter 20 is in place, the conduction valve 420 and the flow adjustment mechanism 430 on the extension tube 410 are turned on in sequence. In this case, the negative pressure injector 510 is connected to the aspiration cavity 111, and the negative pressure injector 510 aspirates blood through the negative pressure. During the aspiration, the aspiration catheter 20 is slowly pushed along a blood vessel to allow an embolus to enter the negative pressure injector 510 with the blood, and vacuum formed by the negative pressure injector 510 disappears until the negative pressure injector 510 is filled with the blood.

During the aspiration, the flow adjustment mechanism 430 on the extension tube 410 can be adjusted according to the situation to control the flow of an aspiration pathway. When withdrawing the aspiration catheter 20, the negative pressure injector 510 is kept in a negative pressure state, to avoid the thrombus or plaque in the aspiration cavity 111 from falling back into the blood vessel. After the aspiration is completed, the conduction valve 420 is closed, and then the aspiration catheter 20 is withdrawn from the blood vessel. If the thrombus is not completely cleared, another negative pressure injector 510 is connected for aspiration again.

As shown in FIG. 13 and FIG. 16, the collection component 600 includes a filter net basket 610 and a waste liquid box 620. The waste liquid box 620 is configured to collect the blood drawn out by the negative pressure injector 510. The waste liquid box 620 includes a box body 621 and a box cover 622 disposed on the box body 621. A baffle plate 623 is disposed at an opening in an end portion of the box body 621, and at least one liquid injection hole 624 is provided in the baffle plate 623. The negative pressure injector 510 injects the blood into the waste liquid box 620 through the liquid injection hole 624, and the baffle plate 623 is configured to prevent the blood injected into the waste liquid box 620 from leaking out from an edge of the waste liquid box 620.

As shown in FIG. 17, the filter net basket 610 is configured to filter out a thrombus. The filter net basket 610 is cylindrical and internally has a space for accommodating a thrombus. A sieve 611 for filtering out a thrombus is disposed at a bottom of the filter net basket 610, and a handle 612 for holding by an operator is disposed at a side surface of the filter net basket 610.

In a specific embodiment, the blood in the negative pressure injector 510 is injected into the waste liquid box 620 through the filter net basket 610. The filter net basket 610 is configured to intercept the aspirated thrombus or another embolus, and the intercepted thrombus or embolus will be left in the filter net basket 610. The filtered thrombus or embolus can be used for subsequent experimental analysis. In this embodiment, the filter net basket 610 is configured to intercept thrombi or other emboli larger than 68 micrometers.

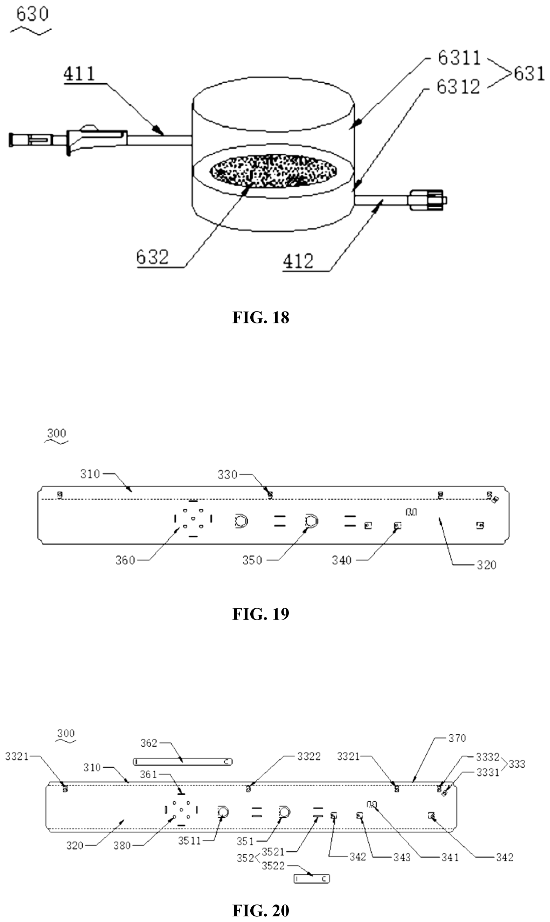

As shown in FIG. 18, in other embodiments, the collection component 600 may further include a filter member 630 disposed on the extension tube 410. The filter member 630 includes a filter box 631 and a filter screen 632 disposed in the filter box 631. The filter box 631 includes a front filter cavity 6311 and a rear filter cavity 6312, and the filter screen 632 is disposed between the front filter cavity 6311 and the rear filter cavity 6312.

The extension tube 410 includes a front tube body 411 and a rear tube body 412. Two ends of the front tube body 411 are respectively connected to the aspiration connection port 212 and the front filter cavity 6311, and two ends of the rear tube body 412 are respectively connected to the rear filter cavity 6312 and the negative pressure source 500. The flow adjustment mechanism 430 is disposed on the front tube body 411 or the rear tube body 412.

In a specific embodiment, on one hand, the filter box 631 is configured to receive the blood exported from the extension tube 410. The blood first enters the front filter cavity 6311. After passing through the filter screen 632, the blood enters the rear filter cavity 6312. The filter screen 632 is configured to intercept the thrombus or another embolus in the blood, to prevent the negative pressure source 500 from being blocked by the thrombus or another embolus in the blood. On the other hand, after the aspiration is completed, a doctor can take out the thrombus or another embolus from the filter box 631 for testing, which provides convenience for postoperative work.

As shown in FIG. 19 and FIG. 20, the assembling base plate 300 includes a catheter assembling area 310 and an accessory assembling area 320. As shown in FIG. 12, the length of the assembling base plate 300 is greater than the length of the aspiration catheter 20. The length dimension of the assembling base plate 300 is greater than its width dimension. The catheter assembling area 310 and the accessory assembling area 320 are respectively located on two sides in a length direction of the assembling base plate 300. The aspiration catheter 20 is disposed in the catheter assembling area 310, and the catheter accessory 30 is disposed in the accessory assembling area 320.

The axial length of the aspiration catheter 20 is much greater than the diameter of an inner cavity of the aspiration catheter. The axial length is usually greater than one meter. Therefore, to compress the size of the aspiration system 10 after packaging, the existing aspiration system 10 is usually packaged by curling and coiling the aspiration catheter 20, so as to reduce the axial length of the aspiration catheter 20 after packaging, thus facilitating transportation.

However, when the aspiration catheter 20 is packaged by using the traditional curling and coiling method, an inner cavity of the catheter body 100 may collapse in a long-term curled and coiled state. Especially for the aspiration catheter 20 designed with two cavities: the guidewire cavity 121 and the aspiration cavity 111. The guidewire cavity 121 and the aspiration cavity 111 of the catheter body 100 are more prone to deformation after being coiled. This can easily cause the guidewire cavity 121 to deform towards the aspiration cavity 111 or cause the aspiration cavity 111 to deform towards the guidewire cavity 121. Over time, plastic deformation may occur between the two cavities of the catheter body 100, which can affect the aspiration rate or the passage of the guidewire, and in severe cases, may cause the product to fail.
Therefore, in the present embodiment , the length of the catheter assembling area 310 of the assembling base plate 300 is greater than the length of the aspiration catheter 20, so that the aspiration catheter 20 can be fully straightened when fixed on the catheter assembling area 310, thereby preventing the assembled catheter body 100 from bending, ensuring the structural integrity of the guidewire cavity 121 and the aspiration cavity 111, and making the inner cavity of the aspiration catheter 20 less susceptible to collapse.

The catheter assembling area 310 includes a catheter fixing component 330 disposed on the assembling base plate 300, and the catheter fixing component 330 is configured to fix the aspiration catheter 20 and avoid bending of the aspiration catheter 20. The catheter fixing component 330 includes a fixed casing 331 for assembling the aspiration catheter 20, a casing fixing member 332 for clamping the fixed casing 331, and a catheter seat fixing member 333 for clamping the catheter seat 200. The casing fixing member 332 and the catheter seat fixing member 333 are respectively disposed on the assembling base plate 300, and the catheter body 100 of the aspiration catheter 20 is threaded into the fixed casing 331 during assembly.

The traditional aspiration catheter is usually fixed directly with a buckle. Although this fixing method is simple, an outer layer of the catheter body 100 of the aspiration catheter 20 is usually coated with a functional coating, such as a hydrophilic coating. During the assembling, disassembling, transportation, and long-term storage of the aspiration catheter 20, there is a risk that the buckle structure of the catheter body 100 scratches off the hydrophilic coating on the catheter body 100.

Therefore, in the present embodiment, after the aspiration catheter 20 is threaded into the fixed casing 331, the casing fixing member 332 is then used to clamp and fix the fixed casing 331 on the assembling base plate 300, thereby avoiding direct contact between the buckle and the catheter body 100, and effectively avoiding the functional coating on the catheter body 100 from being scraped off.

The inner diameter of the fixed casing 331 is greater than the outer diameter of the catheter body 100, so that when the catheter body 100 is threaded into the fixed casing 331, the opening of the fixed casing 331 is less likely to scratch an outer wall of the catheter body 100, which further protects the functional coating on the catheter body 100.

In addition, the fixed casing 331 is in a straight cylindrical shape. An axial direction of the fixed casing 331 is arranged in the length direction of the catheter assembling area 310 of the assembling base plate 300. The hardness of the fixed casing 331 is greater than the hardness of the catheter body 100, so that after the catheter body 100 of the aspiration catheter 20 is plugged into the fixed casing 331, during operation and long-term storage of the aspiration catheter 20, the catheter body 100 can maintain the straight cylindrical state, to avoid the collapse of the aspiration cavity 111 and the collapse of the guidewire cavity 121 due to the bending of the catheter body 100.

The casing fixing member 332 includes two casing fasteners 3321 configured to fasten and fix two end portions of the fixed casing 331, and at least one casing supporting member 3322 disposed at a middle portion of the fixed casing 331 and configured to support the fixed casing 331. The inclination directions of the two casing fasteners 3321 are opposite, so that the fixed casing 331 can be further compressed and fixed. By arranging the casing supporting member 3322 at the middle portion of the fixed casing 331, it is possible to prevent the collapse of the middle portion of the fixed casing 331.

In this embodiment, the two casing fasteners 3321 are respectively inclined towards the middle portion of the fixed casing 331, so that after the casing fasteners 3321 tighten the fixed casing 331, the fixed casing 331 is pressed from two sides towards the middle, making the fixed casing 331 more secure and avoiding relative movement between the fixed casing 331 and the assembling base plate 300. Thus, this avoids friction between the opening of the fixed casing 331 and an outer wall of the catheter body 100 and protects the functional coating on the catheter body 100.

In other embodiments, an anti-skid structure such as an anti-skid coating or anti-skid patterns, may be further disposed on an outer wall of the fixed casing 331 to make the fastening between the fixed casing 331 and the casing fixing member 332 more stable, which further avoids relative movement between the fixed casing 331 and the assembling base plate 300.

In this embodiment, the catheter seat fixing member 333 is disposed at one end of the fixed casing 331, and the catheter seat fixing member 333 is configured to fasten and fix the catheter seat 200. The catheter seat fixing member 333 includes an aspiration fixing member 3331 for clamping the aspiration seat 210 and a guidewire fixing member 3332 for clamping and fixing the guidewire seat 220. The orientation of the guidewire fixing member 3332 is the same as the axial direction of the guidewire seat 220, and the aspiration fixing member 3331 and the guidewire fixing member 3332 are respectively inclined towards the middle portion of the catheter seat 200, thereby clamping and fixing the catheter seat 200 to make the entire aspiration catheter 20 more stable.

When the aspiration catheter 20 needs to be removed from the assembling base plate 300, the catheter seat 200 is first removed from the catheter seat fixing member 333, and then the catheter seat 200 is moved away from the fixed casing 331, so that the catheter body 100 is gradually pulled out of the fixed casing 331 with the movement of the catheter seat 200, and is finally completely withdrawn from the fixed casing 331.

The accessory assembling area 320 includes a connection fixing component 340 for fixing the connection tube component 400, a negative pressure fixing component 350 for fixing the negative pressure source 500, and a collection fixing component 360 for fixing the collection component 600. The accessory assembling area 320 is located at one end of the assembling base plate 300 close to the catheter seat fixing member 333. Since the overall length of the aspiration catheter 20 is usually greater than one meter, if the accessories are scattered, it is not convenient for operation by an operator, and the working efficiency is affected. Therefore, the accessory assembling area 320 is close to the catheter seat fixing member 333, which can make the catheter seat 200 of the aspiration catheter 20 and the catheter accessory 30 distributed in a more concentrated manner. When the operator takes out the aspiration catheter 20 and the catheter accessory 30 from the assembling component 40, it is more convenient for operation.

In this embodiment, since the mass of the negative pressure source 500 is greater than the mass of the collection component 600 and the mass of the connection tube component 400, the negative pressure fixing component 350 of the accessory assembling area 320 is located between the connection fixing component 340 and the collection fixing component 360 to balance the weight applied to the assembling base plate 300 in the length direction. After the catheter accessory 30 is disposed on the assembling base plate 300, the gravity balance point of the assembling base plate 300 is close to the middle area of the assembling base plate 300, which facilitates transportation and storage.

The connection fixing component 340 includes a valve body fixing member 341 for clamping the conduction valve 420, an extension fixing member 342 for clamping the extension tube 410, and an adjustment fixing member 343 for clamping the flow adjustment mechanism 430. In this embodiment, at least two extension fixing members 342 are disposed on the assembling base plate 300 in the length direction of the assembling base plate 300. The two extension fixing members 342 are configured to respectively clamp two ends of the extension tube 410, so that after the extension tube 410 is clamped and connected to the extension fixing members 342, the axial direction of the extension tube 410 is the same as the axial direction of the aspiration catheter 20. The inclination directions of the two extension fixing members 342 are opposite, so that after the extension fixing members 342 clamp the extension tube 410, the extension tube 410 can be further compressed and fixed, making the extension tube 410 assembled more stably.

The adjustment fixing member 343 is arranged between the two extension fixing members 342; and the flow adjustment mechanism 430 is arranged between the adjustment fixing member 343 and one of the two extension fixing members 342. In this embodiment, the flow adjustment mechanism 430 is disposed at one end of the extension tube 410 close to the negative pressure source 500, so as to make the gravity balance point of the assembling base plate 300 further approach the middle area of the assembling base plate 300.

The valve body fixing member 341 is arranged between the catheter assembling area 310 and the extension fixing member 342, specifically between the casing fixing member 332 and the extension fixing member 342. The conduction valve 420 is located between the aspiration catheter 20 and the extension tube 410 when being clamped and connected to the valve body fixing member 341. Consequently, the weight on the assembling base plate 300 in a width direction is balanced, making the gravity balance point of the assembling base plate 300 approach the middle area of the assembling base plate 300, thus facilitating transportation and storage.

The negative pressure fixing component 350 includes a negative pressure sleeve 351 and a negative pressure clamp 352 which are disposed on the assembling base plate 300. One end of the negative pressure injector 510 is clamped to the negative pressure clamp 352, the other end of the negative pressure injector 510 is inserted into the negative pressure sleeve 351. In this embodiment, a through hole 3511 is provided in the negative pressure sleeve 351. During the assembling of the negative pressure injector 510, the injection tube 511 is plugged into the through hole 3511 on the negative pressure sleeve 351, and the negative pressure clamp 352 is disposed between the grip 513 of the injection tube 511 and the push plate 514 of the piston rod 512.

To maintain the cleanliness inside the injection tube 511 during storage of the negative pressure injector 510, a small amount of sealing liquid needs to be sucked into the negative pressure injector 510. If the aspiration system 10 accidentally touches the push plate 514 during transportation and storage, there is a risk that the piston rod 512 may squeeze the sealing liquid in the injection tube 511 out of the injection tube 511 in advance, even if the sealing liquid has a viscosity. In this embodiment, the negative pressure clamp 352 is disposed between the grip 513 of the injection tube 511 and the push plate 514 of the piston rod 512, and the width of the negative pressure clamp 352 is greater than the minimum distance between the grip 513 and the push plate 514, so as to ensure that the piston rod 512 may not squeeze the sealing liquid in the injection tube 511 out of the injection tube 511 in advance due to accidental touch. The minimum distance between the grip 513 and the push plate 514 is the distance between the grip 513 and the push plate 514 after the sealing liquid is sucked into the injector

The negative pressure clamp 352 includes a negative pressure assembling hole 3521 provided in the assembling base plate 300 and a negative pressure ferrule 3522 passing through the negative pressure assembling hole 3521. The width of the negative pressure ferrule 3522 is greater than the minimum distance between the grip 513 and the push plate 514. After passing through the negative pressure assembling hole 3521, the negative pressure ferrule 3522 clamps and fixes the negative pressure injector 510 between the grip 513 and the push plate 514.

The collection fixing component 360 includes a connection assembling hole 361 provided in the assembling base plate 300 and a connection strap 362 passing through the connection assembling hole 361. The collection component 600 is fixed on the assembling base plate 300 through the connection strap 362. Specifically, the width of the connection strap 362 is greater than one-fifth of the width of the collection component 600 to make the collection component 600 more stable. At least two connection straps 362 are arranged on the assembling base plate 300 to fix the collection component 600 in different directions, thus making the collection component 600 fixed more reliably.

Step plates 370 are disposed at edges of two sides of the assembling base plate 300, and the step plates 370 are disposed at a predetermined angle with the assembling base plate 300. The vertical distance from the edges of the step plates 370 to the assembling base plate 300 is at least greater than the thickness of the aspiration catheter 20. Therefore, after the aspiration catheter 20 is assembled on the assembling base plate 300, the step plates 370 can protect the aspiration catheter 20 and other catheter accessories 30 from side surfaces, to prevent damage to the aspiration catheter 20 during transportation and storage. In this embodiment, the predetermined angle between the step plates 370 and the assembling base plate 300 is 90 degrees to 120 degrees, thereby balancing the width dimensions of the step plates 370 and the connection strength.

The assembling base plate 300 is further provided with a ventilation hole 380, and the ventilation hole 380 is configured to sterilize the assembling base plate 300.

By using the above technical solution of the present embodiment, the length of the catheter assembling area 310 of the assembling base plate 300 is greater than the length of the aspiration catheter 20. The aspiration catheter 20 is inserted into the fixed casing 331, so that the aspiration catheter 20 can be fully straightened when fixed on the catheter assembling area 310, thereby preventing the assembled catheter body 100 from bending, ensuring the structural integrity of the guidewire cavity 121 and the aspiration cavity 111, and making the inner cavity of the aspiration catheter 20 less susceptible to collapse.

The foregoing descriptions are merely preferred embodiments of the present disclosure, but are not intended to limit the protection scope of the present disclosure. Any variation or replacement readily figured out by a person skilled in the art within the technical scope disclosed in the present disclosure shall fall within the protection scope of the present disclosure. Therefore, the protection scope of the present disclosure shall be subject to the protection scope of the claims.

## Claims

1. An aspiration system, comprising an aspiration catheter, a catheter accessory, and an assembling component, wherein the assembling component comprises an assembling base plate, and the aspiration catheter and the catheter accessory are detachably connected to the assembling base plate; the assembling base plate comprises a catheter assembling area for assembling the aspiration catheter; the aspiration catheter comprises a catheter seat and a catheter body which are connected to each other; and the length of the catheter assembling area is greater than the length of the aspiration catheter.

2. The aspiration system according to claim 1, wherein the catheter assembling area is provided with a catheter fixing component for fixing the aspiration catheter; the catheter fixing component comprises a fixed casing; and the catheter body is threaded into the fixed casing.

3. The aspiration system according to claim 2, wherein the fixed casing is in the shape of a straight tube and is arranged in a length direction of the catheter assembling area; and the inner diameter of the fixed casing is greater than the outer diameter of the catheter body.

4. The aspiration system according to claim 2, wherein the catheter fixing component further comprises a casing fixing member for clamping the fixed casing; the casing fixing member comprises two casing fasteners respectively for fastening two end portions of the fixed casing; and inclination directions of the two casing fasteners are opposite.

5. The aspiration system according to claim 4, wherein the catheter seat is arranged at a proximal end of the catheter body; the catheter fixing component further comprises a catheter seat fixing member for clamping the catheter seat; the catheter seat fixing member is disposed at one end of the fixed casing; the casing fixing member further comprises at least one casing supporting member disposed at a middle portion of the fixed casing and configured to support the fixed casing;
the catheter seat comprises a guidewire seat connected to the catheter body, and an aspiration seat connected to the catheter body; the catheter seat fixing member comprises an aspiration fixing member for clamping the aspiration seat, and a guidewire fixing member for clamping the guidewire seat; and the aspiration fixing member and the guidewire fixing member are respectively inclined towards a middle portion of the catheter seat.

6. The aspiration system according to claim 1, wherein the assembling base plate further comprises an accessory assembling area for mounting the catheter accessory; and the catheter assembling area and the accessory assembling area are respectively located on two sides along a length direction of the assembling base plate.

7. The aspiration system according to claim 6, wherein the catheter accessory comprises a negative pressure source for providing a negative pressure, and a connection tube component connected between the aspiration catheter and the negative pressure source; and the accessory assembling area comprises a connection fixing component for fixing the connection tube component, and a negative pressure fixing component for fixing the negative pressure source.

8. The aspiration system according to claim 7, wherein the connection tube component comprises an extension tube connected to the aspiration catheter; the connection fixing component comprises at least two extension fixing members for clamping the extension tube; the at least two extension fixing members are disposed in the length direction of the assembling base plate in sequence; and inclination directions of the two extension fixing members are opposite.

9. The aspiration system according to claim 8, wherein the connection tube component further comprises a conduction valve and a flow adjustment mechanism which are disposed on the extension tube; the connection fixing component further comprises a valve body fixing member for clamping the conduction valve and an adjustment fixing member for clamping the flow adjustment mechanism; and the valve body fixing member is disposed between the catheter assembling area and the extension fixing member.

10. The aspiration system according to claim 9, wherein the adjustment fixing member is disposed between the two extension fixing members; and the flow adjustment mechanism is disposed between the adjustment fixing member and one of the two extension fixing members.

11. The aspiration system according to claim 7, wherein the negative pressure fixing component comprises a negative pressure sleeve and a negative pressure clamp which are disposed on the assembling base plate; one end of the negative pressure source is clamped to the negative pressure clamp; and the other end of the negative pressure source is inserted into the negative pressure sleeve.

12. The aspiration system according to claim 11, wherein the negative pressure source comprises a negative pressure injector; the negative pressure injector comprises an injection tube and a piston rod movably connected to the injection tube; the injection tube is provided with a grip; a push plate is disposed at a tail end of the piston rod; the negative pressure sleeve is provided with a through hole for allowing the injection tube to pass through; and the negative pressure clamp is disposed between the grip and the push plate.

13. The aspiration system according to claim 6, wherein the catheter accessory comprises a collection component for collecting waste liquid; the accessory assembling area comprises a collection fixing component for fixing the collection component; the collection fixing component comprises a connection assembling hole provided in the assembling base plate, and a connection strap passing through the connection assembling hole; and the width of the connection strap is greater than one-fifth of the width of the collection component.

14. The aspiration system according to claim 13, wherein the collection component comprises a filter net basket and a waste liquid box; the waste liquid box comprises a box body, a box cover disposed on the box body, and a baffle plate disposed at an opening in an end portion of the box body; at least one liquid injection hole is provided in the baffle plate; the filter net basket is tubular; a filter net is disposed at a bottom of the filter net basket; and a handle is disposed on a side surface of the filter net basket.

15. The aspiration system according to claim 1, wherein step plates are disposed at edges of two sides of the assembling base plate; and the vertical distance from an edge of each step plate to the assembling base plate is at least greater than the thickness of the aspiration catheter.
